# EUROPEAN PATENT APPLICATION

(11) **EP 4 509 038 A1**
(43) Date of publication of application: **19.02.2025**
(21) Application number: 23192265.9
(22) Date of filing: 18.08.2023
(51) Int. Cl.: A61B 5/00, A61B 10/00, G01N 33/493, A47K 11/00, A47K 13/00, E03D 9/00

(54) **SPACER FOR URINE ANALYSIS DEVICE**

(71) Applicant: Withings, 92130 Issy-Les-Moulineaux (FR)
(72) Inventor: Pons, Arthur, 92130 Issy-les-Moulineaux (FR)
(74) Representative: Withings IP

(57) **Abstract**

The invention concerns a urine analysis device comprising a case configured to be positioned entirely within a toilet bowl.

The case comprises a front face for receiving a stream of urine directly from a user urinating in the toilet bowl and a rear face opposite the front face.

The device comprises a collection aperture located on the rear face, the collection aperture being configured to collect liquid inside the case.

The device comprises at least a spacer protruding out of the rear face and configured to be in contact with the toilet bowl.

## Description

### Field of the invention

The present invention relates to a urine analysis device comprising a case configured to be positioned entirely within a toilet bowl.

### Background

Many biological parameters are identifiable in the urine of an individual. For example, health problems such as urinary tract infection, diabetes or kidney failure can be detected from a urine sample. The urine sample can also reflect the quality of a diet, identify a fertile period or pregnancy and detect drug or tobacco use. It is then interesting to monitor various biological parameters periodically.

It is known to offer toilet devices with a urine analysis function. These devices are capable of taking urine samples from the toilet and analyzing them to determine the level of a biological parameter.

Such a urine analysis device is notably described in document WO2021/175909. This document discloses a point-of-care device for urine analysis. The device is to be lodged in a toilet and collects sample of a urine stream before performing an optical analysis. The device comprises a station and a cartridge, which is also called a rotatable support, and which may be removed and replaced from the station. The cartridge contains urinary test strips, that is to say strips coated or impregnated with a reagent that reacts with urine.

This urine analysis device may comprise a collection aperture and a drain aperture to collect the urine on the rear face and evacuate urine. The device may be fixed through a hook mounted on a rim of the toilet bowl. When testing the urine analysis device in real condition, the inventors noticed several issues with the urine collection.

### Summary of the disclosure

More particularly, the inventors have noticed two different issues at collection aperture with this design, notably a limited urine collection in the collection aperture and a problematic recirculation of the drain fluid ejected through the drain aperture and collected again by the collection aperture.

An aim of the disclosure is therefore to provide a urine analysis device solving at least one of the above mentioned issues.

To that end, an aspect of the disclosure relates to a urine analysis device comprising a case configured to be positioned entirely within a toilet bowl, the case comprising a front face for receiving a stream of urine directly from a user urinating in the toilet bowl and a rear face opposite the front face, wherein the urine analysis device comprises a collection aperture located on the rear face, the collection aperture being configured to collect liquid inside the case, and wherein the urine analysis device comprises at least a spacer protruding out of the rear face and configured to be in contact with the toilet bowl.

Thanks to this device, the spacer enables to keep the rear face away from the inside of the toilet bowl and more particularly the region of the rear face in the vicinity of the collection aperture. Therefore, the spacer prevents the collection aperture to be obstructed by the toilet bowl. Indeed, the inventors found out that a bottleneck could exist between the urine analysis device and the toilet bowl, in the vicinity of the collection aperture. This bottleneck may create a reservoir which also collect unwanted fluids (water from flush, liquid splash, evacuated urine, etc.). In one embodiment the spacer enables to detach the urine from the case and may therefore avoid drain recirculation in the collection aperture.

In one implementation, the spacer is arranged so that the collection aperture is at a distance from the toilet bowl in normal use of the urine analysis device.

In one implementation, the collection aperture is arranged near a lower end of the case, in a normal use position of the urine analysis device.

In one implementation, the spacer is located near a lower end of the urine analysis device.

In one implementation, each spacer is arranged next to the collection aperture.

In one implementation, the device comprises two spacers arranged on the rear face on each side of the collection aperture.

In one implementation, the two spacers are symmetrical along a plane, in a normal use position of the device.

In one implementation, the collection aperture is away from each spacer.

In one implementation, each spacer has a fin shape.

In one implementation, each spacer extends from an edge of the rear face towards the collection aperture.

In one implementation, each spacer protrudes on a maximal height of at least 4 mm.

In one implementation, each spacer extends along a main axis forming with a vertical axis, in a normal use position of the urine analysis device, an angle comprised between 0° and 90°, preferably between 60° and 70°, notably around 65°.

In one implementation, the case comprises a drain aperture, separate from the collection aperture, the drain aperture being configured to let liquid out from inside the case.

In one implementation, the drain aperture is arranged on the rear face.

In one implementation, the drain aperture is offset on a side of the rear face.

In one implementation, the drain aperture is arranged on the rear face so that the spacer is configured to guide the liquid coming out of the drain aperture on a ridge of the spacer towards the toilet bowl.

In one implementation, the drain aperture is arranged above the spacer(s) in a normal use position of the urine analysis device.

In one implementation, the drain aperture is arranged on one of the spacers.

In one implementation, the front face is smooth.

In one implementation, the front face is substantially rotationally symmetrical.

In one implementation, the diameter of the case is comprised between 50 mm and 150 mm.

In one implementation, the case is made of hydrophilic material that is one of: a ceramic, a polyamide, a silicone and a hydrophilic polymer.

In one implementation, the case is treated with a hydrophilic surface treatment.

In one implementation, the urine analysis device comprises a test assembly arranged inside the case and configured to perform an analysis on urine collected through the collection aperture.

Another aspect of the disclosure also relates to a urine analysis device comprising a case configured to be positioned entirely within a toilet bowl, the case comprising a front face for receiving a stream of urine directly from a user urinating in the toilet bowl and a rear face opposite the front face, wherein the case comprises a drain aperture, separate from the collection aperture, the drain aperture being configured to let liquid out from the case, wherein the urine analysis device comprises at least a spacer protruding out of the rear face, wherein the drain aperture is arranged on the rear face so that the spacer is configured to guide the liquid coming out of the drain aperture on a ridge of the spacer towards the toilet bowl.

Other features of such spacer were disclosed above, in relation with other aspects of the disclosure.

Another aspect of the disclosure also relates to a urine analysis device comprising a case configured to be positioned entirely within a toilet bowl, the case comprising a front face for receiving a stream of urine directly from a user urinating in the toilet bowl and a rear face opposite the front face, wherein the urine analysis device comprises a collection aperture located on the rear face, the collection aperture being configured to collect liquid inside the case, and wherein the rear face of the case includes an array of ribs, the array of ribs forming at least a portion of a urine pathway from the front face to the collection aperture on the rear face.

In one implementation, each rib comprises a ridge configured to guide urine.

In one implementation, within a toilet bowl means in a volume defined by the toilet bowl.

In one implementation, at least one rib of the array of ribs converges towards the collection aperture.

In one implementation, every rib of the array of ribs converges towards the collection aperture.

In one implementation, at least one rib, for example several ribs of the array of ribs, extends at least partially across the rear face.

In one implementation, at least one rib of the array of ribs extends across the rear face, from an edge of the rear face of the case towards the collection aperture.

In one implementation, wherein a plurality of ribs of the array of ribs extend between a first end arranged on the edge of the rear face and a second end at the vicinity of the collection aperture, wherein the first ends are arranged on an angular range of at least 90° on each side of the rear face

In one implementation, the spacing between two consecutives ribs of the array decreases from a position along the ribs towards the collection aperture.

In one implementation, two consecutive ribs of the array of ribs get closer to one another from a position along the two consecutive ribs towards the collection aperture.

In one implementation, each rib is sensibly straight along the rear face.

In one implementation, at least a rib of the array of ribs extends along a curve.

In one implementation, a plurality of ribs of the array of ribs extend along a curve and a curvature radius of successive ribs of said plurality of ribs of the array of ribs decreases.

In one implementation, at least a rib of the array forms a portion of a circle, preferably centered on a center of the rear face.

In one implementation, the array comprises a plurality of concentric portions of a circle and at least a rib is straight and extends from a center region of the rear face towards the collection aperture.

In one implementation, a height of the ribs of the array decreases starting from a position along the ribs towards the collection aperture.

In one implementation, a height of the ribs of the array of ribs becomes null before the ribs reach the collection aperture.

In one implementation, the collection aperture is not between two rib of the array.

In one implementation, along any urine pathway direction, the collection aperture is at a distance from any rib of the array.

In one implementation, the collection aperture is arranged on a rib of the array of ribs.

In one implementation, the collection aperture is arranged near an edge of the rear face, such as a lower edge.

In one implementation, the collection aperture is arranged near a lower end of the case, in a normal use position of the device.

In one implementation, the array of ribs comprises more than five ribs, notably more than ten ribs.

In one implementation, the maximum height of each rib is comprised between 3 mm and 30 mm, or between 3 mm and 10 mm.

In one implementation, two consecutive ribs form a groove therebetween, and, in a section transversal to the urine pathway, each inflection point between a ridge of a rib and a bottom of the associated groove is closer to the ridge of said rib than the bottom of said groove.

In one implementation, the array of ribs is symmetrical along a vertical plane, in a normal use position of the urine analysis device.

In one implementation, the rear face comprises a fixation pin configurated to cooperate with a fastener configured to fasten the urine analysis device into the toilet bowl.

In one implementation, the fastener is a flexible hook or a suction pad.

In one implementation, the fixation pin is arranged on the upper side of the case, in a normal use position of the urine analysis device.

In one implementation, at least one rib of the array of ribs extends across the rear face, from the fixation pin towards the collection aperture.

In one implementation, the front face is smooth.

In one implementation, the front face is substantially rotationally symmetrical.

In one implementation, the case comprises a drain aperture, separate from the collection aperture.

In one implementation, the drain aperture is arranged on the rear face.

In one implementation, the drain aperture is offset on a side of the rear face.

In one implementation, the diameter of the case is comprised between 50 mm and 150 mm.

In one implementation, the case is made of hydrophilic material that is one of: a ceramic, a polyamide, a silicone and a hydrophilic polymer.

In one implementation, the case is treated with a hydrophilic surface treatment.

In one implementation, the urine analysis device comprises a test assembly arranged inside the case and configured to perform an analysis on urine collected through the collection aperture.

In one implementation, the rear face comprises at least a spacer protruding out of the rear face and configured to be in contact with the toilet bowl. The spacer protrudes higher than the ribs, so that the ribs do not contact the toilet bowl. In addition, thanks to the spacer, the collection aperture may be at a distance from the toilet bowl in normal use of the urine analysis device.

In one implementation, each spacer is arranged in the vicinity to the collection aperture.

In one implementation, the rear face comprises two spacers on each side of the collection aperture.

In one implementation, the collection aperture is away from each spacer.

### Brief description of the Drawings

These features and advantages of the invention will appear more clearly upon reading the following description, provided solely as a non-limiting example, and done in reference to the appended drawings, in which:
Figure 1 shows the overall setup of a urine analysis device according to an embodiment, as installed in a toilet bowl,
Figure 2 shows an exploded view of an embodiment of the urine analysis device, in which the station and the cartridge are visible,
Figure 3 shows a detailed view of a cartridge according to an embodiment,
Figure 4 shows a sectional view of an embodiment of a cartridge and a station, at the location of an optical analyzer of the station,
Figure 5 shows a side view of the urine analysis device according to an embodiment, as installed in a toilet bowl, on an internal surface of the toilet bowl,
Figure 6 shows a rear face of the urine analysis device according to an embodiment,
Figure 7 shows a cross sectional view of the urine analysis device of Figure 5,
Figure 8 shows a perspective view of the rear face of the urine analysis device of Figure 5, in a position corresponding to a position in use, wherein a urine pathway towards a collection aperture of the urine analysis device is illustrated,
Figure 9 shows a perspective view of the rear face of the urine analysis device of Figure 5, in a position corresponding to a position in use, wherein a drain pathway from the drain aperture of the urine analysis device is illustrated,
Figure 10 shows a perspective view of a rear face of a urine analysis device according to another embodiment, in a position not corresponding to a position in use,
Figure 11 shows a perspective view of the rear face of the urine analysis device according to another embodiment, in a position corresponding to a position in use, wherein a drain pathway from the drain aperture of the urine analysis device is illustrated,
Figure 12 shows a perspective view of the rear face of the urine analysis device according to another embodiment, and
Figure 13 shows a graph comparing the collected volume of urine with a urine analysis device of WO'909 and WO'944 and with a urine analysis device according to the present disclosure.

### Detailed description

The present description introduces the principle of a station usable with different cartridges, as disclosed in document WO2021/175909 and WO2021/175944, hereafter referred to as WO'909 and WO'944. Variations of the stations are presented in any of WO2023/036805, WO2023/036806, WO2023/036808, WO2023/036809, hereafter referred to as WO'80X.

The next paragraphs explain the overall principle of a urine analysis device, but all the details of WO'909, WO'944 are applicable.

### ORIENTATION

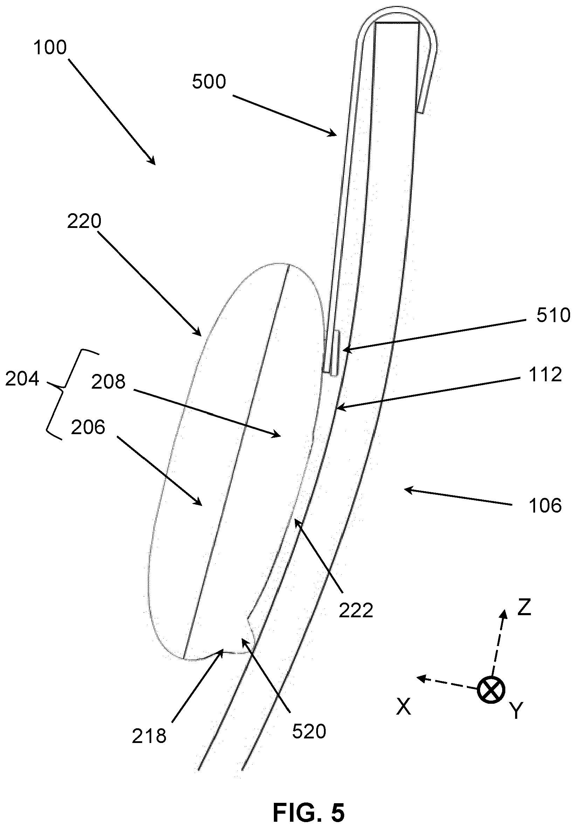

In the following disclosure, the notions of "upper" and "lower", etc. are defined in relation to a Z direction, as defined on Figure 5. The top along the Z direction is defined in a normal use position of the urine analysis device fixed in the toilet bowl.

### OVERALL CASE SHAPE

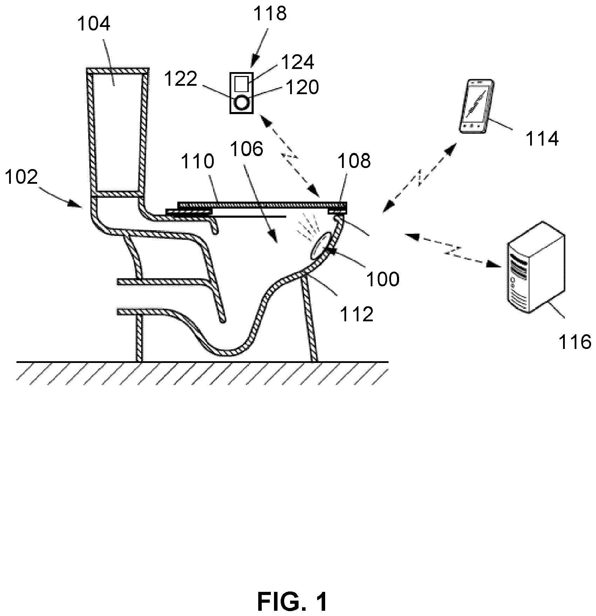

Figure 1 schematically illustrates an analysis device 100 (referred to as the "device 100") for urine analysis as set up in a toilet 102. Toilet 102 usually comprises a water tank 104, a bowl 106, a seat 108 and a seat cover 110. The urine analysis device 100 is configured to be positioned entirely within the toilet bowl. Within a toilet bowl means in a volume defined by the toilet bowl. The analysis device 100 is arranged in a removable manner in the toilet 102. For example, the analysis device 100 may be easily removed from the toilet to replace a cartridge and then arranged again in the toilet 102. The analysis device 100 is arranged on an internal wall 112 of the bowl 106 of the toilet. The analysis device 100 is placed so that it is usually under a urine stream from a user, such that when a user urinates (typically in a seated position), urine contacts with the analysis device 100. The analysis device 100 may communicate remotely with a remote entity, such as smartphone 114 or a server 116.

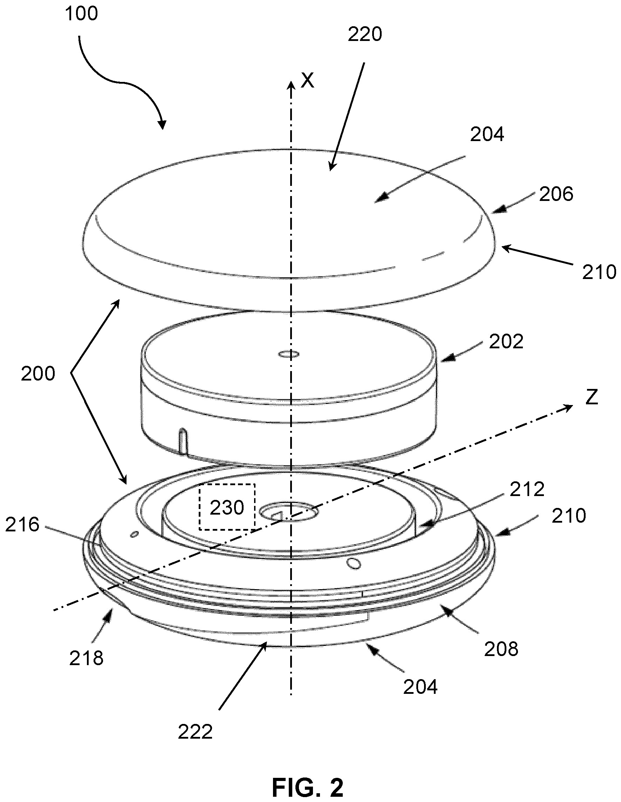

As illustrated in more detail on Figure 2, the analysis device 100 may comprise a station 200 and a cartridge 202, mounted in a removable manner from the station 200. Station 200 may comprise a case 204 which may include two shells, notably a front shell 206 and a rear shell 208. The front shell 206 and the rear shell 208 may cooperate with each other through a fixing mechanism 216, in a plane normal to the axis X. The front shell 206 and the rear shell 208 may be assembled in a reversible way, for example by screwing or clipping. In a variant, the front shell 206 and the rear shell 208 may be assembled in a permanent way, for example by gluing, clipping, magnetizing, or ultrasonic welding. Of course, other fixing means may be used to assemble the two shells.

In particular, as illustrated on Figure 2, the front shell 206 and the rear shell 208 are screwed together. Then, an internal portion of the front shell 206 comprises a thread. The thread of the front shell 206 is intended to cooperate with a complementary thread of the rear shell 208. This allows the case 204 to be easily disassembled for access to the test assembly inside the case.

A seal may be present between the front shell 206 and the rear shell 208. Thus, the case 204 is waterproof. Only the collection and drain apertures connect the exterior and interior of the case 204, as described in more detail below.

As visible on the Figures, the case 204 may have an outer global shape of a circular pebble. In other words, the case 204 has a spheroid shape. An axis X is the centerline of the case. Advantageously, the front shell 206 may be substantially rotationally symmetrical, which gives a streamlined appearance to the device once installed. The case 204 serves as a urine collector.

The case 204 comprises a front face 220 for receiving a stream of urine directly from a user urinating on the toilet and a rear face 222 opposite the front face 220. As illustrated on Figure 2, the front face 220 may be arranged on the front shell 206 and the rear face 222 may be arranged on the rear shell 208. The front face 220 faces the interior of the toilet bowl 106. The front face 220 is then intended to receive urine when the user urinates while sitting on the toilet 102. As visible on Figure 5, the rear face 222 faces the inner wall 112 of the toilet bowl 106. The front face 220 and the rear face 222 comprise each a curved edge 210. The respective curved edge 210 of the front face and the rear face meet flush at an equatorial junction area. Thus, the outer surface of the case 204, consisting of the front face 220 and the rear face 222, is defined by curved lines, and forms a generally convex object.

The outer surface of front face 220 may be smooth. In other words, the front face 220 is devoid of ridges or grooves. Thus, the urine stream contacting the front face 220 clings to and spreads over the front face 220. The front face 220 may be substantially rotationally symmetrical, around the X axis.

The rear face 224 will be described more in detail below.

The outer surface of the case 204 may further be white or light colored. The color of the outer surface may be similar to that of the toilet, increasing the discretion of the device.

The case 204 may have a diameter, measured in the direction normal to the axis X, of between 50 mm and 150 mm. The case 204 may have a thickness, measured in the direction of the axis X, of between 15 mm and 50 mm. Thus, the case 204 is sufficiently compact to be completely received in the toilet bowl. The urine analysis device 100 is discrete. In addition, the case 204 is large enough to systematically come into contact with urine being received in the toilet bowl. The user can then urinate in the toilet without worrying about the urine analysis device, or alternatively aiming summarily.

In another aspect, in one embodiment, the case has a general form factor such that the thickness on diameter ratio is in a range comprised between 0.2 and 0.5, and preferably even in a range comprised between 0.3 and 0.4. Such proportions are reminiscent of a natural pebble and gives the device a soothing appearance.

Preferably, the case 204 is made of a hydrophilic material. For example, the material of the case 204 may be one of: a ceramic, a polyamide (PA), a silicone or a hydrophilic polymer. The outer surface of the case 204 may also be treated with a hydrophilic surface treatment, for example acuWet^{®} from Aculon, a hydrophilic polymer, or Pebax^{®} from Arkema.

### TEST ASSEMBLY

A test assembly 230 is arranged inside the case 204 and configured to perform an analysis on urine collected through the collection aperture. Station 200 comprises an annular or ring-shaped compartment 212, located inside the case 204, arranged around a rotation axis X. The annular compartment 212 is configured to receive at least partially the cartridge 202 mounted in a rotatable manner around the rotation axis X (once in position in the annular compartment 212). The cartridge 202 comprises a plurality of test supports which each comprise at least one urine reagent, for instance a dry reagent, the plurality of test supports being arranged along a circle or a circular arc around the rotation axis X. In an embodiment, the test supports are test strips. The test supports may be enclosed, for example individually enclosed, in a chamber.

The annular compartment 212 typically extends around 360° and forms a groove configured to receive at least partially the cartridge 202.

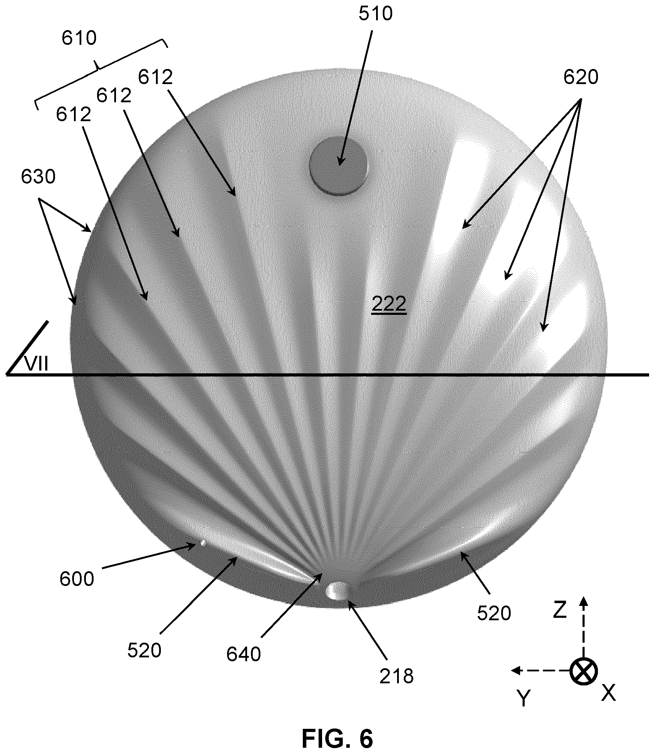

The station 200 comprises a collection aperture 218, located for example on the rear shell 208. As it will be explained more in detail below, the collection aperture 218 is configured to collect urine flowing on the surface of the case 204. The station 200 further comprises a drain aperture 600, visible on Figure 6, configured to drain the liquid out of the device 100.

The test assembly 230 may comprise a pump, an injector, and an analyzer. The pump sucks urine from the collection aperture 218, then the injector injects the urine on one or more test supports of the cartridge and the analyzer obtains some values of properties (e.g., physical/chemical properties, such as the color) of the test supports after it has contacted the urine. In one embodiment, the analyzer is an optical analyzer configured to analyze optical properties of the test support. The injector and the cartridge may move relatively to each other so that the injector can open (e.g., pierce) the chamber, for example with a needle or needle-like device.

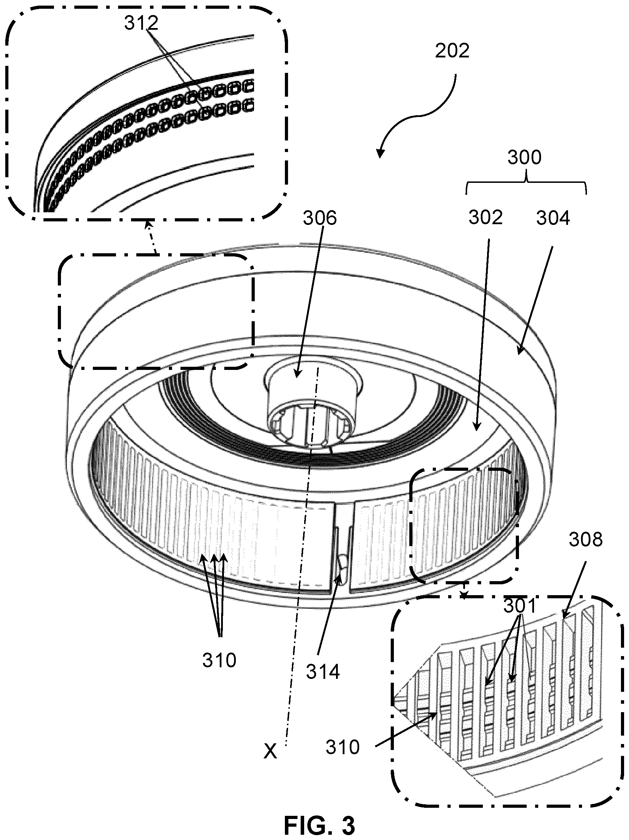

Figure 3 shows an exploded view of the cartridge 202. The cartridge 202 comprises at least a test support 301, notably several test supports 301 configured to receive urine from the injector. Each test support 301 contains a urine reagent that reacts in a specific way when in contact with urine. The cartridge 202 comprises a rotatable support 300, configured to be driven in rotation by the station 200. In normal use of the cartridge 202 and the device 100, the test supports 301 remain attached to the rotatable support and do not move with respect to the latter.

In an embodiment, the rotatable support 300 has a right circular cylinder shape of at least 80% of a hollow cylinder shape extending annularly around an axis which is, when the cartridge 202 is mounted in the station 200, the rotation axis X. Each test support 301 may be a test strip. The rotatable support 300 may comprise an annular portion 302 and a cylindrical portion 304, which extends from an outermost radial extremity of the annular portion 302. The cylindrical portion 304, when in use, is lodged inside the annular chamber 212. The test supports 301 are positioned along the cylindrical portion 304, so that they can be selectively and/or successively scrolled in front of the injector and the analyzer. For instance, the test supports 301 are part of a holder 308, which comprises several chambers 310, separated from each other along a perimeter around the axis X. At least a test strip is received in a chamber 310.

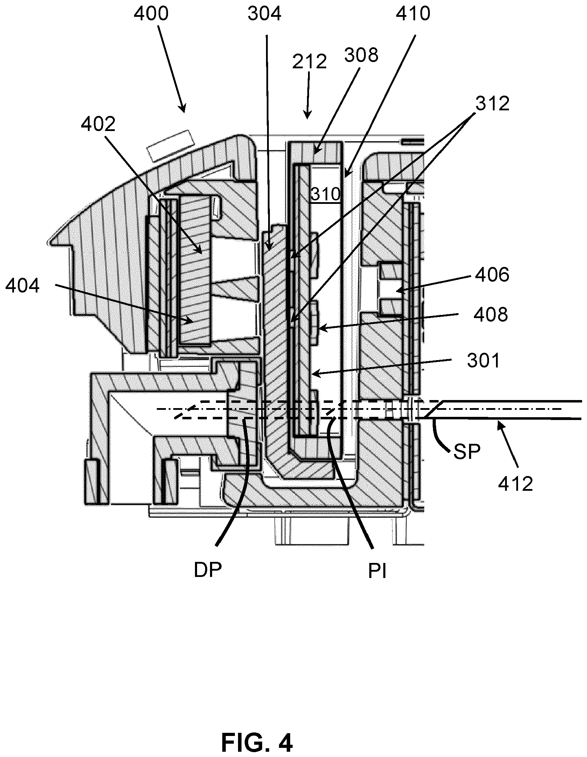

The plurality of chambers 310 are arranged next to one another in a right circular cylinder shape of at least 80% of a circle. To allow light to go through, the holder 308 includes at least one aperture 312 per chamber 310 (represented in the upper left zoom where the rotatable support is shown as transparent). The chambers 310 are all at an equal distance of the rotation axis X, so that the injector can selectively inject urine after the desired chamber is positioned at a desired location facing the injector. The injector may translate towards the chamber 310 and pierce a lid closing the chamber 310 (visible on Figure 4). A drain opening 314 is provided in the rotatable support 300 to allow evacuating urine from the injector to the outside of the device 100, via the drain aperture 600 arranged on the case 204.

The annular portion 302 of the rotatable support 300 remains outside of the annular chamber 212 to strengthen the cylindrical portion and/or to drive in rotation the cartridge 202. To that end, the annular portion 302 may include a mechanical coupling 306, which cooperates with a shaft of the station 200.

The dimensions relative to the cartridge 202 are disclosed in WO'909, WO'933, and WO'80X. The maximum dimension of the device 100 transversal to the rotational axis X is less than 15 cm, even less than 10 cm. The maximum dimension of the device along the rotation axis X is less than 5 cm.

Figure 4 shows in more detail the interaction between the cartridge 202 and the station 200 when or after the injector is activated. The analyzer 400 comprises at least one light source 402, 404 (e.g., two) and at least one optical sensor 406. Light travels from the light source 402, 404 to the optical sensor 406 while passing through the cartridge 202 and in particular the cylindrical portion 304, the aperture 312 of the holder 308, the test support 301 and thus the reagent 408.

In one embodiment, the analyzer 400 is configured to measure the absorbance of a part of the test supports 301 (notably the test line and/or control line of a test strip as it will be explained below). The absorbance is detected by the light source (for example a led) which may pass light through the strip, and the optical sensor which receives the spectrum with about ten wavelengths.

In a variant, the light sensor is a camera able to detect a change of color, notably a change of intensity of color, of a part of the test supports 301 (notably the test line and/or control line of a test strip as it will be explained below). The camera may detect a color in RGB values for example.

The injector includes an injection end 412 (for example a needle), which can be moved between several positions, which are represented in dash lines in Figure 4. In a standby position SP, the injection end 412 is outside the cartridge 202 (in an innermost position), so that the cartridge 202 can rotate freely in the annular housing 212; in an injection position IP, the injection end 412 has pierced the lid 410 to access the inside of the chamber 310 and may inject some urine on the test support 301; in the drain position DP, the injection end 412 is able to evacuate urine through the drain opening 314 of the rotatable support 300.

In position SP, the injector is located radially inward the annular chamber. This allows to maximize the radius of the annular chamber while minimizing the size of the station 200.

In a variant of the above disclosed test assembly 230, the skilled person will understand that every test assembly configured to analyze the collected sample of urine through the collection aperture 218 may be arranged inside the case 204.

### FASTENER

The case 204 is arranged on the toilet internal wall 112 of the toilet bowl 106. The case 204 is fixed by at least a fastener 500. The fastener 500 is configured to fasten the urine analysis device 100 into the toilet bowl.

A fixation pin 510 is arranged on the rear face 222. The fixation pin 510 is configurated to cooperate with the fastener 500. As visible on the Figures, the fixation pin 510 may be arranged on the upper side of the case 204. By "on the upper side of the case", it is understood "on the first quarter along the Z direction starting from the upper end of the case 204".

As illustrated on Figure 5, the fastener 500 may be a flexible hook mounted on a rim of the toilet bowl 106, as well known since decades in the field of toilet cleaning.

In another embodiment (not shown), the fastener 500 may comprise a suction cup intended to cooperate with the internal wall 112 of the toilet.

In another embodiment (not shown), the fastener 500 may comprise magnets (or pieces of ferromagnetic material) intended to cooperate with other magnets arranged on the internal wall 112.

In complement or in variant, the fastener 500 may comprise a ball-and-socket connection, allowing the case 204 to be oriented to increase the likelihood of contact with urine being received in the toilet bowl.

### COLLECTION APERTURE

The collection aperture 218 is configured to receive urine flowing by gravity on the outer surface of the case 204. Urine is collected directly from the front face 220 and the rear face 222 of the case 204.

The collection aperture 218 is an opening configured to collect liquid, so that liquid may enter the urine analysis device. The collection aperture 218 is typically circular, with a diameter preferably between 0.3 mm and 2 mm. The diameter of the collection aperture may be chosen to maximize the volume of urine collected from the outer surface of the case 204.

As visible on the Figures, the collection aperture 218 is located on the rear face 222. Thus, the collection aperture 218 faces the internal wall 112 of the toilets when the urine analysis device 100 is positioned in the toilet. This position allows the collection aperture 218 to be hidden from the user's view by the front face 220 of the case. The front face 220 visible to the user resembles a simple, uniform pebble as already mentioned, without any singular points or holes. Also, it should be noted that this position prevents the introduction of contaminants or elements that could obstruct the test assembly 230.

The collection aperture 218 is located on a lower part of the rear face 222. By "on a lower part of the rear face", it is understood "on the last quarter of the face along the Z direction starting from the lower end of the case 204". The lower end faces the bottom of the toilet bowl 106 when the case 204 is positioned in the toilet bowl. This position corresponds to a normal position of use. This position allows urine to be collected by gravity over the majority of the outer surface of the case 204.

In particular, a distance separating the collection aperture 218 from a lower edge of the case 204 is less than 40 mm, preferably less than 20 mm. As illustrated, according to a particular embodiment, the collection aperture 219 is arranged a few millimeters above the lower edge of the case 204. Alternatively, the collection aperture 218 may be on the lower edge (the lower edge being defined when the device 100 is placed for use in toilet).

The collection aperture 218 may be covered by a mesh filter. The mesh filter is for example oblong in shape covering the collection aperture 218. The average mesh opening of the filter is, for example, 20 microns. The mash filter prevents the introduction of contaminants or elements that may clog the test assembly 230, and filters the urine received in the collection aperture 218. Metal may be used for the filter mesh.

### DRAIN APERTURE

The drain aperture 600 allows the urine analysis device to be purged of excess urine and eventually of cleaning fluid.

In an embodiment, the drain aperture 600 may be separate from the collection aperture 218. The drain aperture 600 may present a circular opening. The drain aperture 600 has a diameter between 0.3 mm and 2 mm.

In an embodiment, as illustrated, the drain aperture 600 is located on the lower end of the case 204. The drain aperture 600 may be arranged on the rear face 222. Thus, the drain aperture 600 faces the internal wall 112 of the toilets when the urine analysis device 100 is positioned in the toilet. This position allows the drain aperture 600 to be hidden from the user's view.

The position of the drain aperture 600 on the rear face 222 may be chosen to prevent recirculation of the drained fluid inside the collection aperture 218 which would lead to contamination of the device. In particular, as visible on Figure 6, the drain aperture 600 is offset on a side of the rear face 222, along the Y axis.

In the embodiment illustrated on Figure 6, the drain aperture 600 may be above the collection aperture 218. In a variant, the drain aperture 600 may be at the same level or below the collection aperture 218.

The drain aperture 600 may be covered by a mesh filter. The mesh filter is for example oblong in shape covering the drain aperture 600. The average mesh opening of the filter is, for example, 20 microns. The filter prevents the introduction of contaminants or elements that may clog the test assembly 230.

In a variant, the drain aperture 600 is not protected by a filter. In particular, it could be considered that due to its arrangement, function and the flow rate it ejects, the drain aperture 600 is not likely to be contaminated.

In another embodiment, not shown, the drain aperture may be the same as the collection aperture. A single port limits the number of openings to the interior of the case 204. Thus, the risk of introducing contaminants or elements that could clog the test assembly 230 is reduced.

### ARRAY OF RIBS

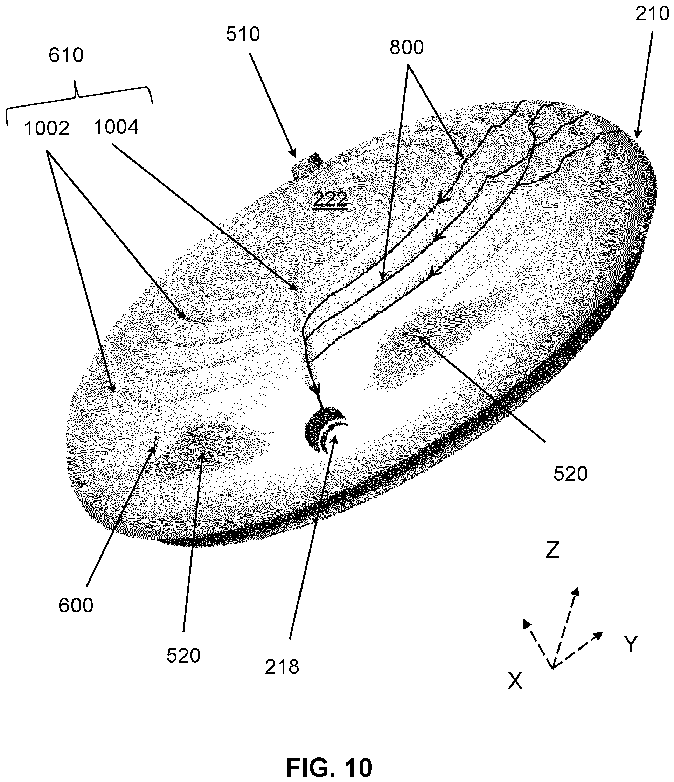

In reference to Figures 6 to 12, the rear face 222 of the case 204 includes an array 610 of ribs 612, 1002, 1004 (referred to as "the array 610"). An array of ribs is a specific arrangement of a plurality of ribs 610 on the rear face 222. The array 610 may comprise more than four ribs, notably more than ten ribs. In one arrangement, the array is symmetrical along. In the illustrated embodiment on Figure 6, the array comprises twelve ribs. In another embodiment illustrated on Figure 10, the array comprises seventeen ribs.

The array 610 forms at least a portion of a urine pathway 800 leading to the collection aperture 218 on the rear face 222. Urine pathway 800 is the path along which the urine is deemed to flow on case 204.

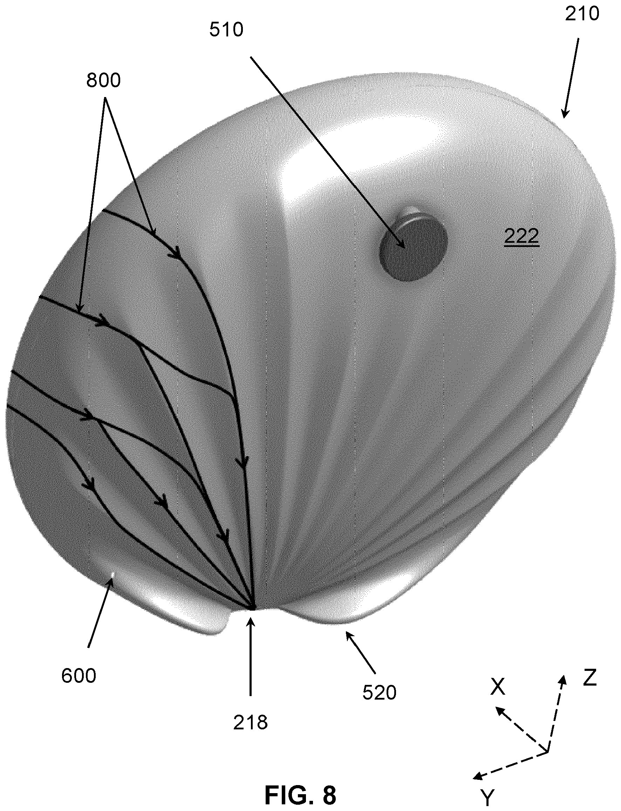

More specifically, each rib of the array 610 may define a portion of the urine pathway 800, so that the array 610 works as a urine collection array. This creates a large urine pathway 800 and thus enables the collection of a large volume of urine that can be brought to the collection aperture 218. Moreover, the array 610 of ribs enables to channel urine towards the collection aperture 218 so that urine is collected continuously. This enables an efficient transit of urine inside the device 100 and a reliable urine injection on the test supports. It has to be borne in mind that, when the urine analysis device is in position in the toilet, the ribs of the array 610 are essentially inclined downward, with the collection aperture 218 at the lower part: urine is therefore guided by the ridge of the ribs and not by the channel between two ribs. In the embodiments of Figures 8 and 10, at least one rib 612, 1004 of the array 610 converges directly towards the collection aperture 218.

The ribs are relief on the rear face 222, with a ridge (which is the apex of the rib) and two channels (or groove) 620 on each side. Two adjacent ribs therefore comprise a channel therebetween. The apex of the ribs (also called "maximum heights") may be comprised between 3 mm and 30 mm, or between 3mm and 10mm. As will be disclosed in further details with at least two embodiments, the distance between two consecutive ribs may be comprised between 5 mm and 50 mm.

### The shell-like arrangement

In reference to Figures 6 to 9 and 11-12, the array 610 of ribs 612 may be arranged in a "shell like" form. The array 610 includes a plurality of ribs 612 converging directly toward the collection aperture 218. Said plurality of ribs may include all the ribs of the array of ribs 610. Each rib 612 of the array 610 extends at least partially across the rear face 222. In other words, each rib 612 is extending inside the rear face 222 and is not only running along the curved edge 210. In one embodiment, each rib 612 of the array 610 extends totally across the rear face 222. Said extension may be carried out along a sensibly straight line such that each rib 612 of the array 610 extends along a sensibly straight direction on the rear face. By straight, it is meant straight disregarding the curvature of the rear face 222. The ribs 612 may be positioned at regular angle intervals from one another (e.g., between 5° and 10°).

The spacing between two consecutives ribs 612 of the array 610 decreases from a position, for example from the edge 210 where the rib 612 starts, along the ribs 612 towards the collection aperture 218. In other words, two consecutive ribs 612 of the array 610 get closer to one another from a position along the two consecutive ribs 612, for example the edge 210, towards the collection aperture 618.

In the embodiment of Figure 8, the array 610 of ribs 612 therefore works as a ramification converging towards the collection aperture 218.

The urine pathway 800 starts from the curved edge 210 of the rear face 222 where the urine is running from the front face 220 and ends at least partially at the collection aperture 218 (partially because not all the urine coming from the urine pathway 800 may be collected). The ribs 612 form a barrier for the urine, slowing the transversal velocity (i.e. transversal to the main direction of the rib)) of the urine and reorienting the urine towards the lower end of the rear face 222 (along the Z axis), especially towards the collection aperture 218. Moreover, as the rear face 222 is inclined downwards in a normal use position of the device 100, gravity pulls the urine to flow on the ridges of the ribs 612, and not in grooves 620 defined between two consecutive ribs 612.

The array 610 of ribs 612 may be symmetrical along a vertical plane XZ, in a normal use position of the device 100, to create a uniform urine pathway.

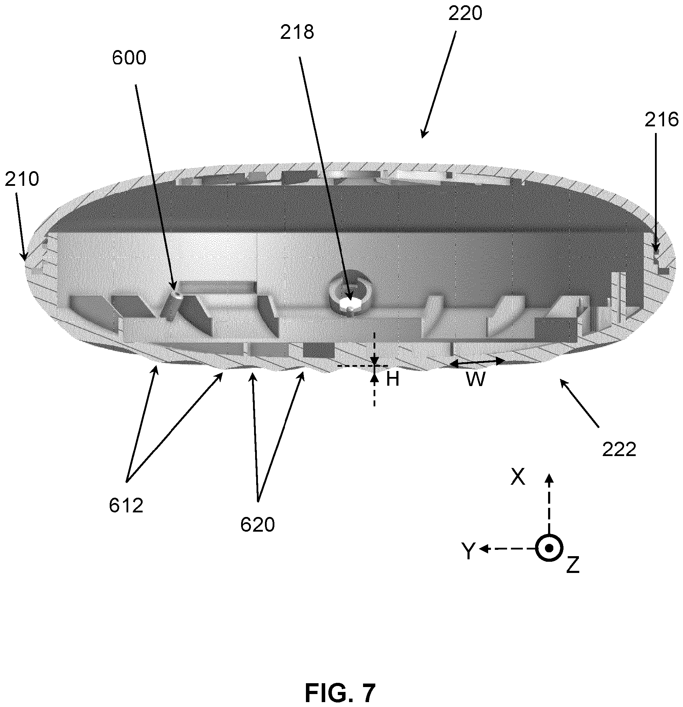

In reference to Figure 7, the maximum height H of each rib 612, defined between the groove 620 and the ridge of said rib 612 (along the X axis or sensibly along the X axis), may be comprised between 3 mm and 30 mm.

In an embodiment, which is illustrated on Figures 6 to 9, a height of the ribs 612 may decrease starting from a position along the ribs 612 towards the collection aperture 218. In particular, starting from the edge 210, the height of the ribs 612 increases until reaching an apex height H and then decreases towards the collection aperture 218. Advantageously, the height of the ribs 612 becomes null before the ribs 612 reaches the collection aperture 218, to enable a smooth junction of the different urine flows before reaching the collection aperture 218. In this embodiment, the collection aperture 218 is at distance from any rib 612 of the array along any urine pathway direction. The distance may be between 5 MM and 20 mm. In particular, the collection aperture 218 is not arranged between two ribs 612 of the array 610.

In a variant, not shown, the collection aperture 218 may be arranged on a rib 612 of the array 610 of ribs 612.

In reference to Figure 7, the maximum width W of each rib 612, defined between the bottom of two consecutives grooves 620, may be comprised between 1 mm and 10 mm.

The ridge of the ribs 612 may be advantageously steep, to better slow down the transversal speed of urine and thus steer urine along the ridge. In particular, in a section transversal to the urine pathway 800, each inflection point in the curvature of the rib 612 between a ridge of said rib 612 and a bottom of the associated groove 620 is closer to the ridge of said rib than the bottom of said groove.

As visible on Figure 6, the array 610 comprises a first plurality of ribs 612 extending across the rear face 222, from the edge 210 towards the collection aperture 218. In particular, the first plurality of ribs 612 extend between a first end 630 arranged on or near the edge 210 of the rear face 222 and a second end 640 at the vicinity of the collection aperture 218.

An angular scale is defined along the edge 210 of the rear face 222, starting at 0° at the lower end of the case 204 and rotating in the clockwise direction, passing by 180° at the upper end of the case 204 and finishing at 360°. As visible on Figure 6, the first ends 630 are arranged on an angular range of at least 90° on each side of the rear face 222. In particular, the first ends 630 may extend regularly between 45° and 160° on the angular scale, and between 200° and 315°.

In an embodiment, some ribs 612 of the array 610 of ribs 612 may extend from the fixation pin 510 towards the collection aperture 218. In particular, two ribs may extend from the fixation pin 510 towards the collection aperture 218.

### The circular arrangement

In reference to Figure 10, the array 610 of ribs 1002 may be arranged in a "concentric circles" form. At least one rib 1002 of the array 610 may extend along a curve, which may be an arc of a circle (e.g., half of a circle or a bit less than half of a circle). As visible on Figure 10, the array 610 may comprise a plurality of ribs 1002 extending along a curve, the curvature radius of the successive ribs 1002 decreasing.

In particular, at least a rib 1002 of the array 610 forms an arc of a circle 1002. The arc of a circle may be centered on a center of the rear face 222. As visible on Figure 10, the array 610 may comprise a plurality of ribs 1002 forms a plurality of concentric portions of a circle. In particular, said plurality of ribs 1002 are centered on the center of the rear face 222.

The plurality of ribs 1002 may comprise a plurality of pairs of arcs of a circle, each rib 1002 of a same pair having the same radius and the same center, but being symmetrical to one another. In the illustrated embodiment, the array comprises eight pair of ribs 1002. Each rib 1002 of the pair is arranged on a respective side of the rear face 222, along the Y axis. Each rib of the pair may extend on an angular range comprised between 120° and 180°.

The arrangement of the ribs 1002 allows to slow down urine flowing transversally to the ribs 1002 and then to guide urine along the ridge of said ribs 1002.

The array 610 of ribs 1002 may also include an additional rib 1004 that converges directly towards the collection aperture 218 and is therefore part of the urine pathway 800, between the ribs 1002 and the collection aperture 218. The additional rib, as illustrated, may be straight and extends from a center region of the rear face 222 towards the collection aperture 218. The ribs 1002 converges towards, and ends before, the additional rib 1004. To that end, the ribs 1002 may extend on an arc of a circle on less than 180°. The additional rib 1004 may end before reaching the collection aperture 218, in a manner similar to the ribs 612 of the shell arrangement disclosed above.

As schematically represented on Figure 10, the urine pathway 800 starts from the edge 210 of the rear face 222 where the urine is coming from the front face 220, follows one of the curved ribs 1002 towards the additional rib 1004, then follows the additional rib 1004 towards the collection aperture 218.

### SPACERS

In reference to Figure 5, in an embodiment, the device 100 comprises at least one spacer 520 protruding out of the rear face 222 and configured to be in contact with the toilet bowl 106, in particular in contact with the internal wall 112 of the bowl 106. The spacer 520 is arranged on the rear face 222. In an embodiment, the spacer 520 is arranged on the rear face 222 so that the collection aperture 218 is at a distance from the toilet bowl 106 in normal use of the device. This prevents the internal wall 112 of the bowl 106 to obstruct the entry of the collection aperture 128 and/or to have a bottleneck for a flow of a mix of urine, water and impurities from the toilet bowl (which could be collected by the collection aperture otherwise).

The spacer 520 enables also to prevent the ribs from touching the toilet bowl 106. For that, the spacer 520 may protrude higher (at least twice) than the ribs (612, 1002, 1004).

Thanks to the at least one spacer 520, the urine flows on the case 502 (particularly the rear face) towards the collection aperture 218 without being bothered by the toilet bowl 106 as it could happen with a device of WO'909 and WO'944. To that end, the fixation pin 510 through which the device 100 may hang from the fastener 500 may also contribute to prevent the ribs from touching the toilet bowl 106. Alternatively (non illustrated), at least one other spacer may be located on the rear (for example in an upper part of the rear face or in a side part of the rear face) to fulfill that function.

In an embodiment, the spacer 520 are located on the lower part of the urine analysis device 100, and more particularly on the lower part of the rear face 222, next to the collection aperture 218.

The spacer 520 typically includes a ridge. When in use, a part of the ridge is contacting the toilet bowl.

In particular, the device 100 may comprise two spacers 520, advantageously arranged on each side of the collection aperture 218, along the Y transversal direction. The two spacers 520 may be symmetrical for example along a vertical plane XZ, in a normal use position of the device 100 or for example around the collection aperture 218.

In an embodiment, the collection aperture 218 is arranged away from each spacer 520. In another embodiment, each spacer 520 may be arranged next to the collection aperture 218. By "next to the collection aperture 218", it is understood at a distance lower than 5 cm, in particular lower than 2 cm.

Each spacer 218 protrudes on a maximal height of at least 4 mm from the rear face 222, notably at least 8 mm.

As illustrated on Figure 5 to 10, each spacer 520 may have a "fin shape", i.e. extending along a length greater than the width, for example two times greater, notably three time greater. In particular, the spacer 520 may present a length comprised between 10 mm and 30 mm. The spacer 520 may present a width comprised between 2 mm and 10 mm. Each spacer 520 may extends from the edge 210 of the rear face 222 towards the collection aperture 218. In particular, starting from the edge 210, the height of the spacer 520 increases until reaching the maximum height and then decreases towards the collection aperture 218. The decrease may be steeper than the increase.

Each spacer 520 may extend along a main axis forming with the vertical axis Z, in a normal use position of the device, an angle comprised between 0° and 90°, preferably between 60° and 70°, notably around 65°.

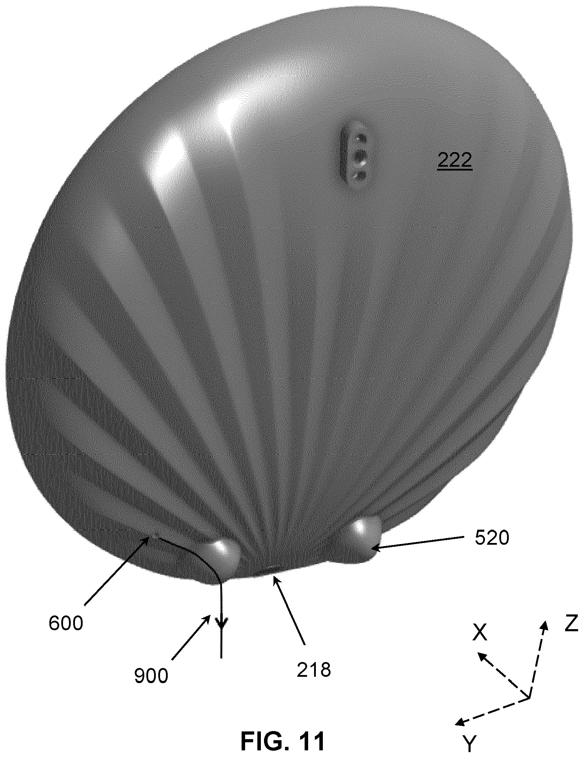

In a variant, illustrated on Figure 11, each spacer 520 may have a "hill shape". In particular, each spacer 520 may present a form of revolution paraboloid. In a variant, not shown, each spacer 520 may present a half ball shape. The spacer 520 may then present a width comprised between 4 mm and 30 mm.

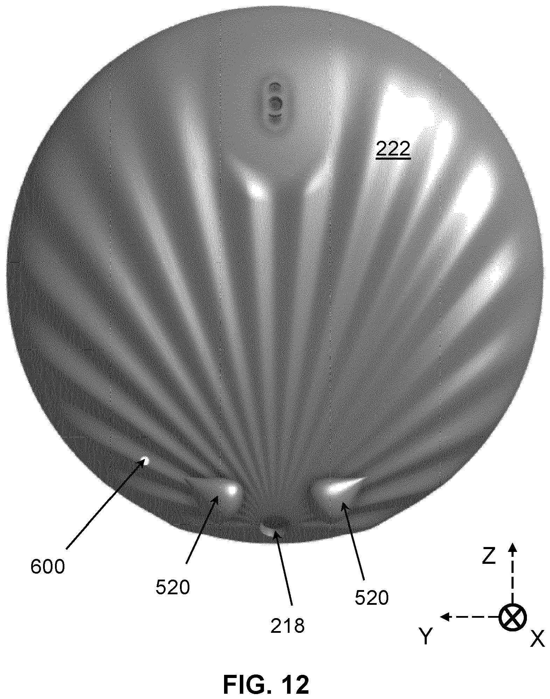

In a variant, illustrated on Figure 12, each spacer 520 may have a "droplet shape". In particular, when moving away from the center along the Y transversal direction, the width of each spacer 520 increases then decreases with a smaller slope until a pointed tip.

In the embodiment illustrated on Figures 5 to 9, the drain aperture 600 may be arranged on one of the spacers 520.

In a variant, as illustrated on Figures 10 to 12, the drain aperture 600 may be arranged above the spacers 520. By above, it is understood upstream of the urine pathway. In particular, the drain aperture 600 may be arranged on, or near, the ridge of a rib 612 converging towards one of the spacers 520.

Another advantage of the spacer 520 will be disclosed here in. It has to be borne in mind that, when the urine analysis device is in position in the toilet, the ribs of the array 610 and the spacer 510 are essentially inclined downward, with the collection aperture 218 being at the lower part.

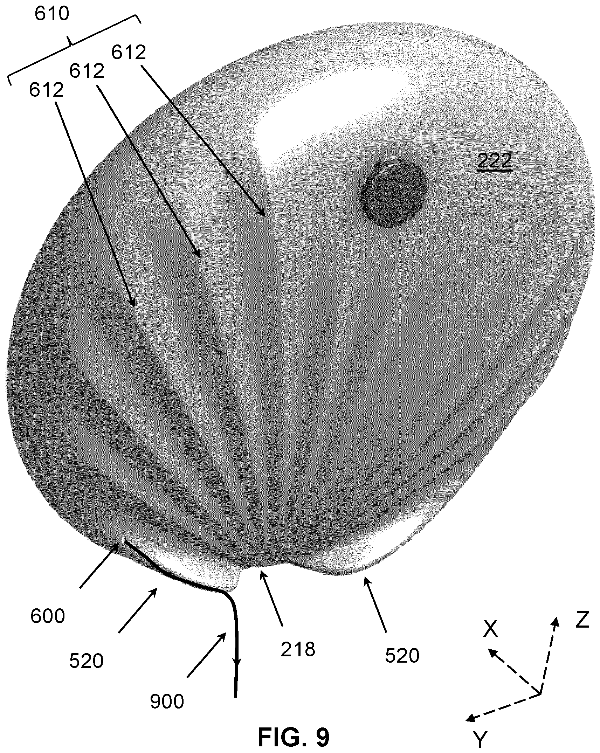

As illustrated on Figure 9 and 11, at least one spacer 520 may form a drain pathway 900 for the drain fluid ejected by the device 100 out of the drain aperture 600. The drain pathway 900 starts from the drain aperture 600. When the drain aperture 900 is arranged on the spacer 520, the drain pathway 900 follows the spacer 520, notably the ridge of the spacer 520 due to gravity. When the drain aperture 900 is above the spacer 520, the drain fluid may be guided by a rib 612 towards the spacer 520 and then flows on the spacer 520. In all the embodiments, the drain pathway 900 then reaches the apex of the spacer 520 and the drain fluid flow on the toilet bowl. The spacers 502 enables therefore the drain pathway 900 not to pass near the collection aperture 218 and so prevent any recirculation and contamination of the drain fluid in the device 100.

As mentioned above, in this embodiment, the drain aperture 900 is located on the spacer 520 or near the spacer so that liquid coming from the drain aperture 900 flows towards the spacer 520. In one implementation, the drain aperture is at the beginning of the fin shape of the spacer 520, that to say at the beginning of the increase of height of the spacer 520.

### TESTS

The inventors carried out tests to compare the collected volume of urine with a urine analysis device of WO'909 and WO'944 and with a urine analysis device according to the present disclosure, in particular with a urine analysis device according to Figure 11.

The protocol of test is the following: a flowrate of water is 15 mL/s is streamed on the front face 220 of the device, then a pumping sequence of 1 minute is triggered. The water stream on the front face is programmed so that it hits three different locations on the front face 220 and leaves the front case in between the hit points. The collected volume at the collection aperture 218 is then measured. The measurements were done three times for each device.

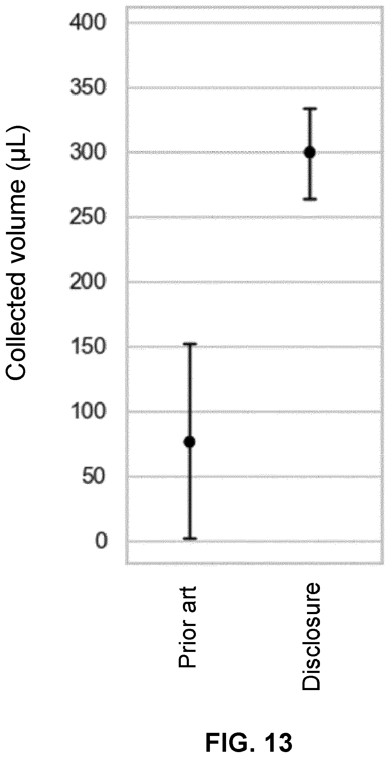

The results are represented on Figure 13. The device according to the device of WO'909 and WO'944 collects in average approximately 75 µL with a wide dispersion in the data. In comparison, the device 100 of the present disclosure collects in average approximately 300 µL with a smaller dispersion in the data. The urine analysis devicec100 of the disclosure is therefore able to collect four times more urine than the device of WO'909 and WO'944 with a similar user miction, with a greater repeatability with essential for a reliable urine analysis.

## Claims

1. A urine analysis device (100) comprising a case (204) configured to be positioned entirely within a toilet bowl (106),
the case (204) comprising a front face (220) for receiving a stream of urine directly from a user urinating in the toilet bowl (106) and a rear face (222) opposite the front face (220),
wherein the device (100) comprises a collection aperture (218) located on the rear face (222), the collection aperture (218) being configured to collect liquid inside the case (204), and
wherein the device (100) comprises at least a spacer (520) protruding out of the rear face (222) and configured to be in contact with the toilet bowl (106).

2. The urine analysis device (100) according to claim 1, wherein the spacer (520) is arranged so that the collection aperture (218) is at a distance from the toilet bowl (106) in normal use of the device (100).

3. The urine analysis device (100) according to claim 1 or 2, wherein the collection aperture (218) is arranged near a lower end of the case (204), in a normal use position of the device (100).

4. The urine analysis device (100) according to any one of claims 1 to 3, wherein the spacer (520) is located near a lower end of the urine analysis device (100).

5. The urine analysis device (100) according to any one of claims 1 to 4, wherein each spacer (520) is arranged next to the collection aperture (218).

6. The urine analysis device (100) according to any one of claims 1 to 5, wherein the device (100) comprises two spacers (520) arranged on the rear face (222) on each side of the collection aperture (218).

7. The urine analysis device (100) according to claim 6, wherein the two spacers (520) are symmetrical along a plane, in a normal use position of the device (100).

8. The urine analysis device (100) according to any one of claims 1 to 7, wherein the collection aperture (218) is away from each spacer (520).

9. The urine analysis device (100) according to any one of claims 1 to 8, wherein each spacer (520) has a fin shape.

10. The urine analysis device (100) according to any one of claims 1 to 9, wherein each spacer (520) extends from an edge of the rear face (222) towards the collection aperture (218).

11. The urine analysis device (100) according to any one of claims 1 to 10, wherein each spacer (520) protrudes on a maximal height of at least 4 mm.

12. The urine analysis device (100) according to any one of claims 1 to 11, wherein the case (204) comprises a drain aperture (600), separate from the collection aperture (218), the drain aperture being configured to let liquid out from inside the case.

13. The urine analysis device (100) according to claim 12, wherein the drain aperture (600) is offset on a side of the rear face (222).

14. The urine analysis device (100) according to claim 13, wherein the drain aperture is arranged on the rear face so that the spacer is configured to guide the liquid coming out of the drain aperture (600) on a ridge of the spacer towards the toilet bowl.

15. The urine analysis device (100) according to any one of claims 12 to 14, wherein:
- the drain aperture (600) is arranged above the spacer(s) (520) in a normal use position of the device (100), or
- the drain aperture (600) is arranged on one of the spacers (520).
